# FASCICULE DE BREVET EUROPEEN

(11) **EP 2 555 667 B1**
(45) Date de publication et mention de la délivrance du brevet: **12.10.2016**
(21) Numéro de dépôt: 11719340.9
(22) Date de dépôt: 07.04.2011
(51) Int. Cl.: A61B 5/00, A61B 3/113

(54) **SYSTEME OPTIQUE DE SUIVI DE MOUVEMENTS OCULAIRES ET DISPOSITIF SUPPORT ASSOCIE**
OPTISCHES SYSTEM ZUR VERFOLGUNG DER AUGENBEWEGUNG UND ZUGEHÖRIGE TRÄGERVORRICHTUNG
OPTICAL SYSTEM FOR FOLLOWING OCULAR MOVEMENTS AND ASSOCIATED SUPPORT DEVICE

(30) Priorité: 09.04.2010 FR 1052720
(43) Date de publication de la demande: 13.02.2013
(73) Titulaire: Suricog, 75015 Paris (FR)
(72) Inventeur: BERNERT, Frédéric, 13013 Marseille (FR); MAILLARD, Mickaël, 92800 Puteaux (FR); KINKINGNEHUN, Serge, 94400 Vitry Sur Seine (FR)
(74) Mandataire: Nony
(86) Numéro de dépôt international: PCT/FR2011/050782
(87) Numéro de publication internationale: WO 2011/124852

(56) Documents cités:
- WO-A1-2008/141460
- WO-A1-2009/043927
- JP-A- 2009 240 551
- US-A- 5 150 137
- US-A- 5 963 300
- US-A- 6 003 991
- US-A- 6 120 461
- US-A1- 2003 030 899
- US-A1- 2006 210 111

## Description

La présente invention concerne un système optique de suivi de mouvements oculaires d'un individu et un dispositif associé de type support porté sur la tête tel qu'un casque.

Le suivi des déplacements oculaires intervient dans de nombreuses applications aussi bien médicales, que technologiques ou marketing.

Par exemple, l'analyse du mouvement des yeux constitue une aide au diagnostic médical, notamment dans le diagnostic de maladies neurologiques et psychiatriques telles que les maladies neurodégénératives incluant les syndromes parkinsonien.

Cette analyse permet également d'approfondir des domaines de recherche, tels que l'exploration visuelle, la stratégie visuelle ou l'exploration de pathologies.

Un autre domaine d'application est la détection des mouvements oculaires pour piloter des systèmes électroniques, comme par exemple le contrôle, par le regard, d'un pointeur informatique ou d'un affichage haute résolution.

Des systèmes optiques traditionnels pour suivre les mouvements oculaires aux fins médicales mettent généralement en oeuvre l'acquisition d'une image d'un ou des yeux d'un patient au moyen d'une ou plusieurs caméras, puis l'analyse de l'image par un dispositif de traitement pour en déduire les mouvements oculaires.

Ces systèmes optiques interposent classiquement un miroir semi-réfléchissant entre les yeux du patient et un sujet qu'il regarde. Ce miroir semi-réfléchissant renvoie, par réflexion, une partie de l'image des yeux vers la ou les caméras disposées hors du champ de vision du patient. C'est par exemple le cas du dispositif décrit dans la publication US 5 150 137.

Certains systèmes optiques utilisent une caméra par oeil. Dans ce cas, la présence des deux caméras rend ces systèmes encombrants et donc difficilement mis en oeuvre de façon portative.

Les systèmes optiques qui utilisent une même caméra pour les deux yeux, doivent, quant à eux, être positionnés à distance des yeux pour pouvoir acquérir une image englobant ceux-ci. Cette distance présente quelques inconvénients, et notamment une plus faible définition (en pixels) allouée à chaque oeil sur l'image et l'introduction de déformations optiques. L'analyse des images pour en déduire les déplacements oculaires s'avère donc plus délicate.

Dans l'ensemble de ces systèmes optiques, la présence d'un miroir semi-réfléchissant dans le champ de vision du patient constitue également un inconvénient notable, à deux titres au moins. D'une part, le miroir semi-réfléchissant peut constituer une zone de référence pour le patient, qui dès lors peut s'en servir comme repère dans des exercices ou tests menés, et donc fausser ces derniers. D'autre part, l'atténuation résultant de la séparation des faisceaux lumineux en deux peut entraîner, pour le patient, une plus grande difficulté à visualiser le sujet regardé, et pour les caméras, le besoin d'une plus grande sensibilité.

Certains dispositifs, plus compacts, ont par ailleurs été développés récemment, permettant leur utilisation portative.

C'est le cas par exemple du dispositif de suivi de mouvements oculaires décrit dans la publication US 2005/280603, concernant en particulier une application de contrôle de la résolution d'affichage, par zones, d'un simulateur de vol.

Le dispositif décrit prend la forme d'un casque prévu pour être posé sur la tête d'un utilisateur. Le casque comprend un viseur semi-réfléchissant monté solidaire du casque et placé dans le champ de vision de l'utilisateur, et une caméra solidaire du casque et placée sur la partie haute du casque hors du champ de vision.

La caméra acquiert une image des yeux, et plus précisément des pupilles, par réflexion simple sur le viseur, ce dernier ayant notamment une face intérieure concave permettant de faire converger, vers la caméra, l'image réfléchie de la zone du visage comprenant les yeux.

Ce dispositif, bien que compact, présente un certain nombre d'inconvénients, et notamment ceux évoqués précédemment en lien avec l'utilisation d'un miroir semi-réfléchissant.

En outre, la concavité de la face intérieure de ce dernier introduit des déformations optiques importantes, et une perte de définition (en pixels) des images des yeux par rapport à l'utilisation d'une caméra par oeil. Il est donc nécessaire, pour obtenir un suivi précis des mouvements oculaires, d'avoir recours à des traitements correctifs adaptés, au niveau du dispositif de traitement.

La présente invention vise à pallier au moins un des inconvénients de l'état de la technique.

Dans ce contexte, l'invention concerne notamment un système optique de suivi des mouvements oculaires d'un visage d'individu, comprenant un module d'acquisition d'images agencé pour acquérir une image des deux yeux de l'individu, caractérisé en ce qu'il comprend un moyen optique de transmission, vers le module d'acquisition, de deux images de respectivement chaque oeil qui correspondent ensemble à une région discontinue du visage par réflexions multiples selon deux trajets optiques dépourvus de miroir semi-réfléchissant. Dans le système optique selon l'invention, les trajets optiques comprennent des chemins optiques d'entrée au regard respectivement de chaque oeil, qui sont parallèles entre eux et à un axe optique du module d'acquisition d'images, et le moyen optique de transmission est configuré pour rapprocher lesdites deux images de sorte que le module d'acquisition acquière simultanément lesdites deux images.

Le système optique selon l'invention offre, à l'aide d'un seul module d'acquisition, des définitions (en pixels) sensiblement équivalentes aux systèmes utilisant deux caméras. Par définition, le système est notamment interposé entre les yeux de l'individu et le module d'acquisition.

Cette définition améliorée est obtenue par la transmission d'images correspondant à une région discontinue du visage et par le rapprochement de ces images pour leur acquisition par le module dédié à cet effet. Ainsi, la zone du visage qui n'est pas transmise, généralement la zone de discontinuité située entre les deux yeux, n'est pas acquise. Les pixels traditionnellement utilisés pour l'acquisition de cette zone entre les deux yeux sont désormais alloués à la définition des zones non contiguës entourant les yeux, grâce à un rapprochement sur une partie de la discontinuité. La définition de ces zones est par conséquent améliorée.

Le rapprochement est notamment réalisé selon un axe parallèle à celui défini par les deux yeux, c'est-à-dire généralement un axe horizontal.

Le système optique selon l'invention est par ailleurs léger car ne comprenant qu'un seul module d'acquisition pour les deux yeux.

Le système optique selon l'invention est également peu volumineux car le moyen optique de rapprochement permet de s'affranchir de la grande distance traditionnellement nécessaire entre le module d'acquisition et les yeux.

Le système optique selon l'invention peut donc avantageusement être mise en oeuvre de façon portative, par exemple au niveau d'un casque prévu pour être porté par la tête de l'individu.

Les images des deux yeux acquises par le système optique selon l'invention sont transmises, de façon classique, à un dispositif de traitement, lequel, par analyse, va en déduire les mouvements oculaires de l'individu.

L'invention telle que définie ici peut trouver application dans les nombreux domaines tels que précédemment évoqués: médical, technologique, marketing, etc.

Dans un mode de réalisation de l'invention, ledit moyen optique de transmission rapproche lesdites deux images par réflexions multiples à l'aide de miroirs plans. Cette disposition permet de limiter les déformations optiques introduites par le rapprochement. En effet, les miroirs plans conservent les dimensions des objets réfléchis.

Toutefois, l'invention n'est pas limitée à l'utilisation de miroirs plans, et des miroirs non plans peuvent être mis en oeuvre pour autant que leur agencement permette de multiples réflexions projetant les images des deux yeux simultanément sur le module d'acquisition d'image.

En particulier, le moyen optique de transmission comprend deux ensembles optiques pourvus chacun d'une zone d'entrée à placer en regard de chaque oeil, et chaque ensemble optique comprend un premier miroir en regard de la zone d'entrée correspondante et un deuxième miroir en regard desdits premier miroir et module d'acquisition d'images. Dans cette configuration, le système optique permet de capturer des zones de haute définition autour de chaque oeil et de les rapprocher optiquement pour qu'elles soient acquises simultanément par un même module d'acquisition.

Bien que l'invention ne soit pas limitée à cette configuration, cette dernière présente une complexité réduite pour assurer l'efficacité de l'invention. Notamment, les miroirs sont configurés pour réfléchir, sensiblement à 90°, les rayonnements lumineux. Cet angle est adapté en particulier au cas où le moyen optique de transmission est configuré pour rapprocher des chemins optiques (au niveau des zones d'entrée) qui sont sensiblement parallèles en conservant leur parallélisme lorsqu'ils sont projetés sur le module d'acquisition. La conservation de ce parallélisme garantit l'absence de déformation optique sur les images acquises des yeux.

Selon une caractéristique particulière, lesdits deuxièmes miroirs des deux ensembles optiques sont contigus. Cette disposition offre une optimisation de la surface de détection que constitue le module d'acquisition. En effet, l'image acquise comprend dans ce cas, de façon contiguë, les deux portions non contiguës du visage acquises. Aucun pixel de détection n'est donc perdu. Il en résulte, bien entendu, une définition optimale des portions d'image acquises pour chaque oeil.

En particulier, le bord contigu desdits deuxièmes miroirs est placé sensiblement dans l'axe optique du module d'acquisition d'images. De la sorte, la surface de détection est équitablement répartie pour l'acquisition des portions d'image pour les deux yeux. En outre, cette configuration minimise les déformations optiques des images acquises.

Dans un mode de réalisation, les trajets optiques comprennent des chemins optiques de sortie frappant ledit module d'acquisition d'images, qui sont parallèles entre eux et à l'axe optique du module d'acquisition d'images

Dans un mode de réalisation, le système optique comprend, au niveau de deux zones d'entrée du moyen optique de transmission à placer en regard de chaque oeil, un moyen de filtrage optique de longueurs d'onde. Cela permet de filtrer les signaux lumineux entrant dans le système au niveau des zones d'entrée, pour notamment ne conserver que les longueurs d'onde efficacement détectées par le module d'acquisition. On évite ainsi d'éventuelles interférences dans l'acquisition de l'image des deux yeux.

De façon similaire, le système optique comprend, au niveau de deux zones d'entrée du moyen optique de transmission à placer en regard de chaque oeil, un moyen de polarisation des signaux lumineux entrant dans le système au niveau des zones d'entrée. Cette disposition permet de réduire les reflets présents sur les yeux de l'individu. Il en résulte une détection et une analyse plus efficace des mouvements oculaires.

Dans un mode de réalisation, le système optique est dépourvu de miroir semi-réfléchissant agencé pour propager une image d'un oeil vers le module d'acquisition et une image d'un sujet vers les yeux de l'individu, l'une par transmission à travers le miroir semi-réfléchissant et l'autre par réflexion sur le miroir semi-réfléchissant. Dans ce cas, aucun miroir semi-réfléchissant n'est positionné sur le chemin optique entre les zones d'entrée et le module d'acquisition d'images.

En l'absence de miroir semi-réfléchissant, le système selon l'invention présente un poids réduit et n'est pas placé directement dans le champ de vision de l'individu. Le système optique dispose donc d'une meilleure visibilité du sujet regardé. Comme on le verra par la suite, le système optique selon l'invention peut être placé juste en dessous du champ de vision principal de l'individu.

Selon une caractéristique de l'invention, le système optique comprend des moyens d'éclairement infrarouge pour éclairer, chacun, de façon directe l'un des deux yeux, lesdits moyens d'éclairement fonctionnant en émission continue. Grâce à la non-nécessité d'utiliser un miroir semi-réfléchissant dans l'invention (lequel miroir semi-réfléchissant introduit une atténuation substantielle de la luminosité captée) et à la proximité du module d'acquisition avec les yeux, la présente invention permet d'utiliser efficacement des diodes infrarouge de moindre intensité. Il en résulte la possibilité d'émettre en continu et d'exposer plus longtemps l'utilisateur pour suivre ses mouvements oculaires, sans risque pour lui.

Selon une caractéristique de l'invention, le système optique est monté articulé sur un bras de fixation. Cette disposition permet notamment, lorsque l'individu prend une position prédéfinie relativement au bras de fixation (par exemple un emplacement prévu pour sa tête), d'ajuster l'orientation du système optique pour que les deux zones d'entrée soient mises précisément en regard des deux yeux de l'individu. Cette articulation est donc notamment selon un axe horizontal, parallèle à l'axe formé par les deux yeux.

L'invention a également trait à un dispositif support, par exemple de type casque, prévu pour être placé sur la tête d'un individu ayant deux yeux regardant un sujet dans un plan de vision, comprenant un système optique de suivi de mouvements oculaires comme défini ci-dessus, et dans lequel un axe optique principal du système optique forme une angle avec le plan de vision. Cet angle assure le décalage du système optique par rapport au champ de vision de l'individu. Ainsi, plus cet angle est important, meilleur est le champ de vision pour l'individu. Toutefois, lorsque cet angle prend des valeurs trop importantes, l'image acquise peut être plus ou moins déformée.

Ainsi, selon une caractéristique particulière, l'angle entre l'axe optique principal et le plan de vision est compris entre 20° et 45°, le système optique étant placé en dessous du plan de vision. Il est ainsi possible à la fois d'ajuster le dispositif à la morphologie de l'individu, mais également d'ajuster le champ de vision laissé libre à celui-ci. Compte tenu de la dimension du système optique, cette plage de valeurs permet de laisser un champ de vision libre vers le bas d'un angle sensiblement compris entre 15° et 40°. De préférence, l'angle entre l'axe optique principal et le plan de vision sera choisi de telle sorte que l'angle du champ de vision vers le bas sera d'environ 20°, offrant un bon compromis entre acquisition non déformée des yeux et largeur du champ de vision.

Dans un mode de réalisation, le système optique est monté sur un bras de fixation articulé par rapport à une structure support, de sorte à faire varier l'angle entre l'axe optique principal et le plan de vision. Un ajustement du casque à la morphologie de l'individu est donc possible. Cet ajustement combiné notamment avec le réglage en rotation du système optique par rapport au bras de fixation (tel qu'évoqué précédemment) permet d'obtenir un alignement efficace des zones d'entrée du système optique avec les yeux de l'utilisateur, quelque soit la morphologie de l'individu.

D'autres particularités et avantages de l'invention apparaîtront encore dans la description ci-après, illustrée par les dessins ci-joints, dans lesquels :
- la **figure 1** représente un dispositif de type casque embarquant un système optique selon l'invention;
- la **figure 2** représente, en vue du dessus, un mode de réalisation d'un système optique selon l'invention;
- la **figure 3** illustre de façon schématique le principe de rapprochement optique selon l'invention;
- la **figure 4** illustre une alternative au schéma de la **figure 3****;** et
- la **figure 5** illustre la mise en oeuvre de l'invention pour l'acquisition d'une image des yeux d'un individu, en comparaison avec les solutions de l'état de l'art.

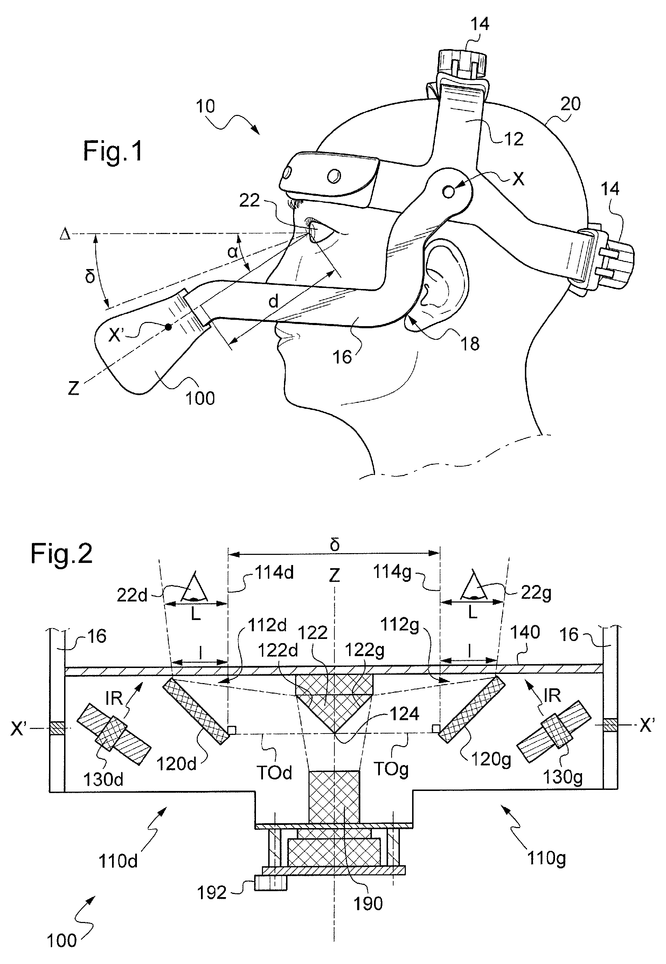

En référence à la **figure 1****,** un support de type casque 10 pour le suivi des mouvements oculaires d'un individu comprend une structure de casque 12 prévue pour être ajustée sur la tête d'un individu 20, par exemple à l'aide de moyens élastiques (bande élastique de maintien faisant le tour de la tête) ou de moyens de réglages mécaniques 14.

Le casque 10 comprend en outre deux bras de fixation 16 de part et d'autre du visage de l'individu, montés mobiles en rotation autour de l'axe horizontal X par rapport à la structure 12.

Les bras de fixation 16 sont coudés et s'étendent, en diagonale, depuis les points de rotation situés à hauteur des zones temporales de l'individu vers le bas du visage, d'abord par une section sensiblement verticale, puis par une section horizontale. Le coude 18 permet, comme le montre la figure, de libérer le champ de vision latéral de l'individu.

A l'extrémité des deux bras de fixations est disposé, de façon articulée selon l'axe horizontal X', un système optique 100 de suivi des mouvements oculaires selon l'invention. Le système optique 100 présente un axe optique principal Z, qui lorsque le système est en utilisation, est sensiblement aligné avec les yeux 22 de l'individu.

Le système optique 100 peut tourner autour de l'axe X' sur une amplitude angulaire d'environ 10°, par rapport à une position initiale pour aligner l'axe optique Z du système 100 avec les yeux de l'individu. Cet alignement permet notamment de bien centrer l'image des yeux sur un module d'acquisition des images prévu dans le système optique.

Les bras de fixation 16 peuvent, quant à eux, tourner autour de l'axe X entre une position où l'axe optique principal Z du système 100 forme un angle a avec un plan de vision Δ (ici horizontal - plan formé par les yeux et un sujet regardé) de l'individu égal à 20° et une position où cet angle est de 35°. Dans l'exemple de la figure, les valeurs de cet angle sont à comprendre avec le système optique 100 situé en dessous du plan de vision Δ. Cette liberté de rotation permet un ajustement vertical du système optique 100 relativement à l'individu.

Cette plage de valeurs [20°, 45°] permet notamment de positionner le système optique 100 de telle sorte que le champ de vision de l'individu vers le bas (angle δ défini entre le plan de vision Δ et le haut du système 100) présente un angle δ compris entre 15° et 40°. Cet angle dépend bien entendu des dimensions du système optique 100.

En particulier, on choisira un angle α telle que l'angle δ soit compris entre 18° et 30°, et notamment égal à 20°, optimisant le ratio entre l'ouverture angulaire du champ de vision vers le bas et la déformation des images des yeux.

Comme illustré sur la figure, la longueur des bras de fixation 16 est prévue pour que la face avant (la plus proche de l'individu) du système optique de suivi de mouvements oculaires soit disposée à une distance d des yeux, comprise entre 7 et 10 cm, et notamment égale à 8 cm.

Le système optique 100 de suivi des mouvements oculaires a pour fonction d'acquérir une séquence d'images des deux yeux de l'individu afin qu'une analyse soit effectuée pour déterminer le comportement des yeux (mouvements oculaires).

Le dispositif de traitement (non représenté) pour réaliser cette analyse peut être embarqué, en tout ou partie, dans le système optique 100. De préférence toutefois, afin de limiter la complexité du système optique 100, ce dispositif de traitement est en majorité extérieur au casque 10.

Par exemple, seuls des traitements relatifs à la détection de la pupille et/ou à l'horodatage des données d'image peuvent être réalisés de façon embarquée. Dans le premier cas, moins d'informations (que les images entières) sont transmises au dispositif de traitement externe. Dans le deuxième cas, cela permet d'assurer la synchronisation entre les images acquises et des stimuli visuels présentés à l'individu, pour corriger d'éventuels délais ou asynchronismes entre le système 100 et le dispositif de traitement.

Ainsi, les données recueillis par le système optique 100 et transmises au dispositif de traitement externe peuvent être de différents types, et notamment des images ou des données de position de chaque oeil ou des données de vitesse, etc.

Lorsque le dispositif de traitement est en majorité extérieur au casque, des moyens de transmission, soit filaires via un connecteur prévu à cet effet au niveau du système optique 100 (192 sur la **figure 2**), soit par ondes (par exemple wifi, bluetooth ou équivalent), sont prévus pour transmettre les données d'images. Le système optique 100 est ainsi relié au dispositif de traitement pour le suivi de mouvements oculaires, qui reçoit ces données d'image. Comme de tels moyens de transmission, ainsi que le dispositif de traitement, sont largement connus de l'homme de l'art, ils ne seront pas décrits plus en détail ici et peuvent prendre la forme d'un ou plusieurs microcontrôleurs ou processeurs.

A noter toutefois que l'acquisition réalisée ici ayant la forme d'images, les traitements opérés pour déterminer le comportement oculaire de l'individu sont par exemple des analyses d'images par détection des yeux (ou pupilles) et par suivi des formes détectées d'image en image.

Bien que la **figure 1** présente l'invention dans un contexte portatif de casque, le système optique 100 peut être monté mobile à l'aide d'un bras de fixation 16 à un dispositif fixe sur lequel est présenté le visage de l'individu à analyser.

La **figure 2** illustre de façon détaillée, en vue du dessus, un mode de réalisation d'un système optique 100 selon l'invention.

Le système optique 100 comprend deux ensembles optiques 110d et 110g associés respectivement aux yeux de l'individu, et disposés sur un même support (non représenté) du système 100.

L'oeil droit 22d est présenté en regard de la zone d'entrée 112d du premier ensemble optique 110d, alors que l'oeil gauche 22g est présenté en regard de la zone d'entrée 112g du deuxième ensemble optique 110g. La mise en regard des yeux avec les zones d'entrée est ajustée notamment au travers de la rotation du système optique 100 autour de l'axe X'.

Les deux ensembles optiques 110d et 110g coopèrent ensemble pour transmettre les images des yeux obtenues au niveau des zones d'entrée 112d et 112g vers un module d'acquisition 190 et pour les rapprocher lorsqu'elles ressortent de ces ensembles optiques de sorte à ce qu'elles soient acquises simultanément par le module d'acquisition 190, de type caméra telle que des capteurs CCD ("*Charge-Coupled Device*") CMOS ("*Complementary metal oxide semi-conductor*") bien connus de l'homme de l'art. La simultanéité ressort ici du fait que les images de chaque oeil atteignent ensemble le capteur (de façon juxtaposée spatialement). Donc l'acquisition à un instant t d'une image par ce capteur comprend l'acquisition des images de chaque oeil.

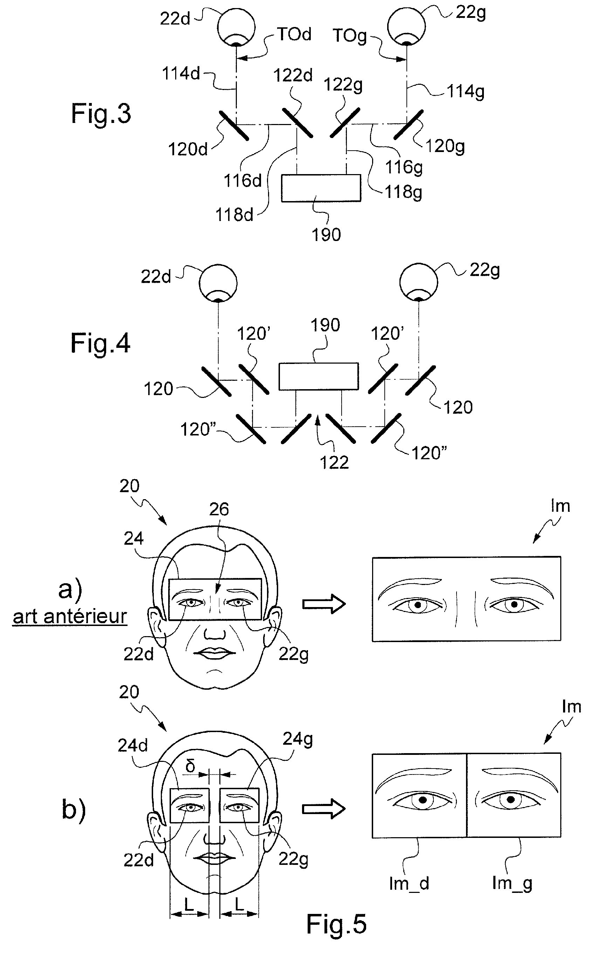

Comme on le verra par la suite en référence à la **figure 5****,** les deux zones d'entrées sont en regard de deux zones non contiguës du visage. Ainsi, les deux images transmises, chacune par l'un des ensembles optiques, correspondent à une région discontinue du visage de l'individu.

Pour effectuer une analyse du suivi des mouvements oculaires très précise, on privilégiera notamment l'acquisition d'images à haute fréquence, par exemple à 100 Hz ou plus et notamment à au moins 300 Hz.

Les zones d'entrée 112d et 112g ne sont pas nécessairement matérialisées par des ouvertures (généralement rondes) pratiquées dans un boîtier du système optique 100 et définissent donc des zones particulières du système eu égard aux chemins optiques décrits ci-après.

Comme illustré schématiquement sur la **figure 3****,** le principe du rapprochement des images dans ce mode de réalisation met en oeuvre des réflexions multiples, en particulier deux réflexions sur des miroirs plans.

L'image de chaque oeil 22d ou 22g acquise parcourt un trajet optique TOd ou TOg qui est composé d'un chemin optique d'entrée 114d ou 114g, un chemin optique intermédiaire 116d ou 116g et un chemin optique de sortie 118d ou 118g.

Les chemins optiques d'entrée 114d et 114g sont définis entre les yeux 22d et 22g et un premier miroir plan 120d ou 120g dans chaque ensemble 110d et 110g. Les chemins optiques d'entrée sont de préférence parallèles entre eux, et par ailleurs parallèles à l'axe optique Z du système optique 100.

Les chemins optiques intermédiaires 116d et 116g relient chaque premier miroir plan 120d ou 120g à un second miroir plan 122d ou 122g, selon les principes de réflexion optique.

Enfin, les chemins optiques de sortie 118d et 118g relient les seconds miroirs 122d et 122g et la caméra 190.

L'agencement des miroirs montré ici est tel que les chemins optiques de sortie sont également parallèles entre eux et parallèles à l'axe optique Z. Cela résulte de l'utilisation de miroirs positionnés à 45° par rapport à chaque chemin optique, de telle sorte que les rayonnements lumineux le long de ces chemins optiques sont réfléchis à 90°.

L'invention s'applique toutefois à d'autres positionnements des miroirs, pour autant que les multiples réflexions projettent les images des deux yeux simultanément sur la caméra 190. En outre, l'angle d'ouverture de la caméra peut faire varier légèrement ces chemins optiques.

Une caméra de faible focale, par exemple 8 mm, peut être utilisée pour offrir une compacité importante. Dans ce cas, lors de la conception du système optique, un ajustement fin de la mise au point de la caméra 190 est réalisée, pour tenir compte des erreurs de positionnement des miroirs dans le système optique. A noter que les variations dues aux formes variables des visages des individus peuvent entraîner de faibles flous dans l'acquisition des images des yeux, sans toutefois entraver l'efficacité des algorithmes de détection de pupilles et/ou de suivi de mouvements oculaires.

Comme montré sur la **figure 2****,** en fonctionnement, les premiers miroirs plans 120d ou 120g sont situés en face des deux yeux 22d et 22g. Les zones d'entrée sont donc les zones avant ces premiers miroirs.

Les premiers miroirs peuvent notamment être formés d'un revêtement réfléchissant à base d'or afin d'obtenir des propriétés de réflectivité maximales dans le spectre visible proche de l'infrarouge et dans l'infrarouge, notamment un coefficient de réflectivité de l'ordre de 96% dans la zone 750-1500 nm. Toutefois, d'autres revêtements moins efficaces (revêtement métallique type aluminium ou argent) peuvent être utilisés, le système optique 100 étant alors moins lumineux.

Comme ces miroirs ne sont pas dans la zone principale (zone d'intérêt) de vision de l'individu 20, il n'ont pas besoin d'être semi-réfléchissant (c'est-à-dire laissant quand même traverser une partie du rayonnement lumineux). Par conséquent, on utilise des miroirs qui ne sont pas semi-réfléchissants, c'est-à-dire des miroirs dans le sens classique du terme. Pour la suite, l'absence de la précision "semi-réfléchissant" indique qu'il s'agit d'un tel miroir pris dans son sens classique, avec une propriété de réflexion totale (ou quasi-totale).

L'image de chaque oeil 22 est, dans l'exemple, réfléchie sensiblement à 90° par le premier miroir 120 pour être projetée sur le deuxième miroir plan 122 qui est typiquement un miroir triangulaire dont deux faces adjacentes contiguës 122d et 122g, positionnées à angle droit, constituent les deux seconds miroirs des ensembles optiques 110d et 110g. De préférence, ces miroirs présentent également un revêtement réfléchissant à base d'or.

Un tel miroir triangulaire permet de rendre contiguës les deux images des deux yeux après leur réflexion sur ce miroir. Comme il ressort de la figure, le rapprochement des images de chaque oeil est fonction de l'éloignement des deux miroirs 122d et 122g ou de l'éloignement de la zone "utile" de ces miroirs utilisée pour la réflexion des images de chaque oeil. Ainsi, selon la réalisation souhaitée, les deux miroirs peuvent être légèrement éloignés pour procurer un rapprochement incomplet des images, ou les zones "utiles" peuvent être décalées par rapport au bord contigu 124, par exemple en décalant (selon l'axe Z vers les yeux) le premier miroir 120 par rapport au second miroir 122.

En particulier, le côté contigu 124 du miroir triangulaire est placé dans l'axe optique (confondu avec l'axe Z) de la caméra 190. Ainsi, les deux images des yeux contiguës sont détectées équitablement (chacune une moitié de la zone de détection) par la caméra.

Ainsi configuré, le miroir triangulaire 122 permet de renvoyer l'image de chacun des yeux sur la moitié du capteur de la caméra.

Les chemins optiques proches de cet axe Z suivent, dans l'exemple de la **figure 2****,** des réflexions à angle droit de telle sortie que les chemins optiques 114d/114g correspondant sont parallèles. L'écart δ entre ces chemins optiques parallèles correspond à la largeur de la zone de discontinuité entre les yeux qui n'est pas acquise par la caméra. Bien entendu, si les deux seconds miroirs 122 ne sont pas contigus, ce parallélisme n'est d'autant pas conservé que la caméra 190 présente un angle d'ouverture important. Dans ce cas l'écart δ est nettement accru.

On notera ici qu'aucun miroir semi-réfléchissant n'est nécessaire dans la présente invention pour procéder au suivi des mouvements oculaires, contrairement aux dispositifs connus.

Sur le schéma de la **figure 2****,** la longueur projetée "l" de ces miroirs sur la vitre 140 protégeant le système optique est de quelques centimètres, par exemple entre 1,4 et 2 cm.

En utilisant une caméra 190 disposant d'un angle d'ouverture approprié, cette longueur projetée "l" délimitant substantiellement ce que la caméra 190 va acquérir, correspond à une zone du visage autour de chaque oeil qui présente une largeur "L" de l'ordre de 5 à 7 cm (compte tenu de la distance "d" d'environ 8 cm avec le visage). Ainsi, le système optique 100 selon l'invention est tolérant à une variation de la distance inter-yeux d'un individu à l'autre.

A noter que cet angle d'ouverture permet d'utiliser des seconds miroirs 122 de plus faibles dimensions que les premiers miroirs 120.

Des relations géométriques classiques pour l'homme de l'art relient tout ou parties des dimensions des miroirs, de la focale et de l'angle d'ouverture de la caméra, de la distance "d", des largeurs "l" et "L" et/ou des distances séparant les miroirs entre eux ou avec la caméra. Ainsi de simples calculs permettent, selon les besoins, de déterminer l'un ou l'autre de ces paramètres (et donc choisir éventuellement les équipements idoines) en fonction des autres.

Bien que la figure montre une caméra disposée du côté opposé du système optique 100 par rapport aux yeux 22, elle peut être prévue du même côté que les yeux en inversant le sens du miroir triangle **(****figure 4****)** où des miroirs supplémentaires 120' et 120" sont prévus pour ajouter de multiples réflexion.

De retour à la **figure 2****,** une diode à rayonnement infrarouge 130d ou 130g est prévue dans chaque ensemble optique 110 pour éclairer, de façon directe, l'oeil correspondant. De façon bien connue en soi, cet éclairage infrarouge permet d'améliorer la détection des yeux dans les images acquises par la caméra 190, grâce à un rehaussement du contraste de l'oeil. Bien entendu, cette caméra est par nature choisie comme détectant dans les longueurs d'onde du visible mais également dans l'infrarouge correspondant auxdites diodes (généralement un infrarouge proche du visible, par exemple une diode à 830 nm).

En raison principalement de l'absence de miroir semi-réfléchissant atténuant les images acquises, mais également en raison de la faible distance du système optique 100 avec les yeux 22, l'éclairement infrarouge par les diodes 130 peut être de faible intensité et en continu comparé aux dispositifs connus (éclairement de plus forte intensité et pulsé en raison de risques liés à l'exposition des yeux à l'éclairement). Le dispositif selon l'invention peut donc avantageusement être utilisé en continu plus longtemps sur un individu, sans risque pour les yeux.

De façon optionnelle, une vitre 140 équipe le système optique 100 au niveau des zones d'entrée 112, entre les yeux de l'individu et les premiers miroirs 120. En variante, deux vitres peuvent être utilisées, placées chacune au niveau d'une des zones d'entrée.

L'utilisation d'une vitre 140, filtrante ou non, permet de protéger physiquement le système optique 100 (de poussières entrantes par exemple).

En outre, on peut choisir une vitre 140 filtrant une partie du spectre visible tout en atténuant que faiblement la lumière de la bande spectrale émise par les diodes. La vitre filtrante 140 est alors notamment choisie pour laisser passer les fréquences les mieux détectées par la caméra 190 et/ou les fréquences correspondant aux couleurs à partir desquelles les traitements subséquents (détection des contours, des pupilles, etc.) sont facilités, notamment le spectre visible proche de l'infrarouge et l'infrarouge à partir.

A titre d'exemple, on peut alors appliquer, sur les images obtenues au niveau de la caméra 190, un algorithme de détection de la pupille par seuillage du noir si le contraste est suffisant et/ou un algorithme de segmentation de la pupille et/ou un algorithme basé sur la recherche de modèles de pupilles.

En variante ou en combinaison, la vitre 140 peut également être choisie polarisante, afin d'éliminer ou atténuer les reflets sur la cornée des yeux 22.

Toutefois, les moyens de filtrage et de polarisation peuvent être prévus distincts, par exemple à l'aide de deux vitres superposées.

Comme il ressort de la description de ce mode de réalisation, l'invention permet notamment d'utiliser une seule caméra filmant indépendamment chacun des yeux. La caméra est placée près des yeux, ce qui permet d'éviter des déformations optiques substantielles de l'image des yeux sur le capteur de la caméra et permet d'améliorer la définition en pixels de l'image acquise pour chaque oeil.

La **figure 5** illustre le rapprochement des images indépendantes de chaque oeil par comparaison avec l'art antérieur connu **(****figure 5a****).**

Dans l'art antérieur, l'utilisation d'une seule caméra, fait qu'une seule zone continue 24 du visage de l'individu 20 est acquise en image 'Im'. Cette zone continue 24 comprend les zones relatives aux deux yeux 22d et 22g mais également la zone 26 du visage située entre les deux yeux de l'individu.

Avec l'invention **(****figure 5b****),** deux zones 24d et 24g du visage qui sont indépendantes, non contiguës et, chacune, centrée sur un des deux yeux, sont acquises par la caméra 190. Comme montré sur la figure, ces deux zones correspondent à une région discontinue du visage de l'individu et le système 100 projette cette région discontinue en une image continue sur le capteur de la caméra 190. Ainsi, le système optique 100 selon l'invention évite l'acquisition de la zone 26 entre les deux yeux car celle-ci n'est pas réfléchie par les premiers miroirs 120d et 120g.

En variante, une partie de la zone 26 peut être réfléchie par les premiers miroirs, mais non transmise jusqu'au module d'acquisition en raison par exemple de l'absence de réflexion par les seconds miroirs 122d et 122g. Pour réaliser cette absence de réflexion, ces seconds miroirs peuvent être de dimensions réduites et disposés de telle sorte à ne réfléchir qu'une sous-partie de ce qui a été réfléchi par les premiers miroirs. Cette sous-partie est alors la zone 24d ou 24g, selon l'ensemble optique considéré. Dans ce cas, la non transmission de la zone 26 résulte de l'absence de réflexion en partie par les premiers miroirs et en partie par les seconds miroirs.

Le système optique 100 selon l'invention permet donc de rapprocher optiquement les images (Im_d et Im_g) relatives aux deux zones 24d et 24g pour qu'elles soient détectées, par la caméra, de façon rapprochée. Ce rapprochement est réalisé sur tout ou partie de la discontinuité entre les deux zones, c'est-à-dire sur tout ou partie de la zone 26 entre les deux yeux. Ainsi, "de façon rapprochée" signifie que la distance entre les deux yeux sur le capteur de la caméra est inférieure à la distance entre les deux yeux sur le visage après transformation par le système optique (en effet le système optique peut insérer une légère modification des distances, même si l'utilisation de miroirs plans permet de limiter celle-ci). En d'autres termes, les deux yeux sur l'image obtenue par l'invention sont plus rapprochés que s'ils avaient été obtenus par un même système en acquérant toute la zone du visage y compris la zone 26 entre les yeux.

En particulier, comme montré sur la figure, les deux images détectées Im_d et Im_g sont contiguës, grâce notamment à l'utilisation de miroirs 122d et 122g contigus (miroir triangle).

Ce rapprochement optique permet de réduire la distance optique entre la caméra 190 et les yeux 22 par rapport aux longues distances requises dans nombre de systèmes de l'état de l'art, de telle sorte que l'image 'Im' des yeux présente une meilleure définition pour chaque oeil. En effet, les pixels traditionnellement utilisés pour l'acquisition de la zone 26 entre les deux yeux sont désormais alloués à l'acquisition d'images Im_d et Im_g des zones 24d et 24g centrées sur les yeux.

Les exemples qui précèdent ne sont que des modes de réalisation de l'invention qui ne s'y limite pas.

En particulier, bien qu'il a été décrit un système optique 100 disposé en dessous de la direction de vision de l'individu, le système optique 100 selon l'invention peut être positionné au dessus de cette direction (obtention de 15° à 40° d'angle de vision vers le haut selon la rotation effectuée des bras 16), mais également sur un côté du champ de vision de l'individu, permettant selon les contraintes imposées de dégager un maximum de champ de vision dans des directions privilégiées.

## Revendications

1. Système optique (100) de suivi des mouvements oculaires d'un visage d'individu (20), comprenant un module (190) d'acquisition d'images agencé pour acquérir une image (Im) des deux yeux (22, 22d, 22g) de l'individu, comprenant un moyen optique de transmission (110d, 110g), vers le module d'acquisition (190), de deux images (Im_d, Im_g) de respectivement chaque oeil qui correspondent ensemble à une région (24d, 24g) discontinue du visage par réflexions multiples selon deux trajets optiques (TOd, TOg) **caractérisé en ce que** les deux trajets optiques sont dépourvus de miroir semi-réfléchissant,
et **dans lequel** les trajets optiques comprennent des chemins optiques d'entrée (114d, 114g) au regard respectivement de chaque oeil, qui sont parallèles entre eux et à un axe optique (Z) du module d'acquisition d'images (190), et
le moyen optique de transmission est configuré pour rapprocher lesdites deux images de sorte que le module d'acquisition (190) acquière simultanément lesdites deux images (Im_d, Im_g).

2. Système optique (100) selon la revendication 1, dans lequel ledit moyen optique de transmission (110d, 110g) rapproche lesdites deux images par réflexions multiples à l'aide de miroirs plans (120, 122).

3. Système optique (100) selon la revendication 1 ou 2, dans lequel le moyen optique de transmission comprend deux ensembles optiques (110d, 110g) pourvus chacun d'une zone d'entrée (112d, 112g) à placer en regard de chaque oeil (22d, 22g), et chaque ensemble optique comprend un premier miroir (120d, 120g) en regard de la zone d'entrée correspondante (112d, 112g) et un deuxième miroir (122d, 122g) en regard desdits premier miroir (112d, 112g) et module d'acquisition d'images (190).

4. Système optique (100) selon la revendication 3, dans lequel lesdits deuxièmes miroirs (122d, 122g) des deux ensembles optiques (110d, 110g) sont contigus.

5. Système optique (100) selon la revendication 4, dans lequel le bord contigu (124) desdits deuxièmes miroirs (122d, 122g) est placé sensiblement dans l'axe optique (Z) du module d'acquisition d'images (190).

6. Système optique (100) selon l'une des revendications précédentes, dans lequel les trajets optiques (TOd, TOg) comprennent des chemins optiques de sortie (118d, 118g) frappant ledit module d'acquisition d'images (190), qui sont parallèles entre eux et à l'axe optique (Z) du module d'acquisition d'images.

7. Système optique (100) selon l'une des revendications précédentes, comprenant, au niveau de deux zones d'entrée (112d, 112g) du moyen optique de transmission (110d, 110g) à placer en regard de chaque oeil (22d, 22g), un moyen de polarisation (140) des signaux lumineux entrant dans le système au niveau des zones d'entrée.

8. Système optique (100) selon l'une des revendications précédentes, comprenant des moyens (130d, 130g) d'éclairement infrarouge pour éclairer, chacun, de façon directe l'un des deux yeux (22d, 22g), lesdits moyens d'éclairement fonctionnant en émission continue.

9. Système optique (100) selon l'une des revendications précédentes, dans lequel le système optique (100) est monté articulé sur un bras de fixation (16).

10. Dispositif (10) support prévu pour être placé sur la tête d'un individu (20) ayant deux yeux (22, 22d, 22g) regardant un sujet dans une plan de vision (Δ), comprenant un système optique (100) de suivi de mouvements oculaires selon l'une des revendications précédentes, et dans lequel un axe optique principal (Z) du système optique (100) forme une angle (α) avec le plan de vision (Δ).

11. Dispositif (10) selon la revendication 10, dans lequel l'angle (α) entre l'axe optique principal (Z) et le plan de vision (Δ) est compris entre 20° et 45°, le système optique étant placé en dessous du plan de vision (Δ).

12. Dispositif (10) selon la revendication 10 ou 11, dans lequel le système optique (100) est monté sur un bras de fixation (16) articulé par rapport à une structure support (12), de sorte à faire varier l'angle (α) entre l'axe optique principal (Z) et le plan de vision (Δ).

## Patentansprüche

1. Optisches System (100) zur Verfolgung okularer Bewegungen eines Gesichts eines Individuums (20), umfassend ein Modul (190) zur Bildaufnahme, das angeordnet ist, um ein Bild (lm) von zwei Augen (22, 22d, 22g) des Individuums zu erfassen, umfassend ein optisches Übertragungsmittel (110d, 110g) zum Erfassungsmodul (190) von zwei Bildern (lm_d, lm_g) von jeweils jedem Auge, die zusammen einer diskontinuierlichen Region (24d, 24g) des Gesichts entsprechen, durch mehrfache Reflexionen gemäß zweier optischer Verläufe (TOd, TOg), **dadurch gekennzeichnet, dass** die zwei optischen Verläufe ohne halbdurchlässige Spiegel sind,
und **wobei** die optischen Verläufe die optischen Eingangspfade (114d, 114g) in Bezug jeweils auf jedes Auge umfassen, die untereinander und zu einer optischen Achse (Z) des Bilderfassungsmoduls (190) parallel sind, und das optische Übertragungsmittel konfiguriert ist, um die zwei Bilder anzunähern, sodass das Erfassungsmodul (190) die zwei Bilder (lm_d, lm_g) gleichzeitig erfasst.

2. Optisches System (100) nach Anspruch 1, wobei das optische Übertragungsmittel (110d, 110g) die zwei Bilder durch mehrfache Reflexionen mittels ebener Spiegel (120, 122) annähert.

3. Optisches System (100) nach Anspruch 1 oder 2, wobei das optische Übertragungsmittel zwei optische Einheiten (110d, 110g) umfasst, die jeweils über einen Eingangsbereich (112d, 112g) verfügen, die in Bezug auf jedes Auge (22d, 22g) anzubringen sind, und jede optische Einheit einen ersten Spiegel (120d, 120g) in Bezug auf den entsprechenden Eingangsbereich (112d, 112g) und einen zweiten Spiegel (122d, 122g) in Bezug auf die ersten Spiegel (112d, 112g) und das Bilderfassungsmodul (190) umfasst.

4. Optisches System (100) nach Anspruch 3, wobei die zweiten Spiegel (122d, 122g) der zwei optischen Einheiten (110d, 110g) aneinandergrenzend sind.

5. Optisches System (100) nach Anspruch 4, wobei der angrenzende Rand (124) der zweiten Spiegel (122d, 122g) im Wesentlichen in der optischen Achse (Z) des Bilderfassungsmoduls (190) angeordnet ist.

6. Optisches System (100) nach einem der vorherigen Ansprüche, wobei die optischen Verläufe (TOd, TOg) optische Ausgangspfade (118d, 118g) umfassen, die auf das Bilderfassungsmodul (190) treffen, die untereinander und zu einer optischen Achse (Z) des Bilderfassungsmoduls parallel sind.

7. Optisches System (100) nach einem der vorherigen Ansprüche, umfassend auf Ebene von zwei Eingangsbereichen (112d, 112g) des optischen Übertragungsmittels (1110d, 110g), die in Bezug auf jedes Auge (22d, 22g) anzubringen sind, ein Polarisationsmittel (140) von Lichtsignalen, die auf Ebene der Eingangsbereiche einfallen.

8. Optisches System (100) nach einem der vorherigen Ansprüche, umfassend Mittel (130d, 130g) zur Infrarotbeleuchtung, die jeweils das eine von zwei Augen (22d, 22g) erhellen, wobei die Beleuchtungsmittel mit kontinuierlicher Abstrahlung funktionieren.

9. Optisches System (100) nach einem der vorherigen Ansprüche, wobei das optische System (100) gelenkig an einem Befestigungsarm (16) montiert ist.

10. Haltevorrichtung (10), die vorgesehen ist, um am Kopf eines Individuums (20) angebracht zu werden, dessen zwei Augen (22, 22d, 22g) ein Subjekt auf einer Sichtebene (Δ) betrachten, umfassend ein optisches System (100) zur Verfolgung okularer Bewegungen nach einem der vorherigen Ansprüche, und wobei eine optische Hauptachse (Z) des optischen Systems (100) einen Winkel (α) mit der Sichtebene (Δ) bildet.

11. Vorrichtung (10) nach Anspruch 10, wobei der Winkel (α) zwischen der optischen Hauptachse (Z) und der Sichtebene (Δ) zwischen 20° und 45° beträgt, und das optische System unterhalb der Sichtebene (Δ) angebracht ist.

12. Vorrichtung (10) nach Anspruch 10 oder 11, wobei das optische System (100) an einem Befestigungsarm (16) gelenkig in Bezug auf eine Trägerstruktur (12) montiert ist, um den Winkel (α) zwischen der optischen Hauptachse (Z) und der Sichtebene (Δ) zu variieren.

## Claims

1. An optical system (100) for tracking the ocular movements of the face of an individual (20), said system comprising an image acquisition module (190) designed to acquire an image (Im) of the individual's two eyes (22, 22d, 22g),
comprising an optical means (110d, 110g) for transmission, to the acquisition module (190), of two images (Im_d, Im_g) of each eye respectively, which together correspond to a discontinuous region (24d, 24g) of the face, by multiple reflections along two optical paths (TOd, TOg) **characterized in that** the two optical paths do not include a semi-reflective mirror,
**and in which** the optical paths comprise optical input paths (114d, 114g) relating to each eye respectively, which run parallel to one another and to an optical axis (Z) of the image acquisition module (190), and
the optical transmission means is configured to bring said two images closer together so that the acquisition module (190) acquires said two images (Im_d, Im_g) simultaneously.

2. An optical system (100) according to Claim 1, in which said optical transmission means (110d, 110g) brings said two images closer together by multiple reflections using plane mirrors (120, 122).

3. An optical system (100) according to Claim 1 or 2, in which the optical transmission means comprises two optical assemblies (110d, 110g), each provided with an input zone (112d, 112g) to be placed opposite each eye (22d, 22g), and each optical assembly comprises a first mirror (120d, 120g) opposite the corresponding input zone (112d, 112g) and a second mirror (122d, 122g) opposite said first mirror (112d, 112g) and said image acquisition module (190).

4. An optical system (100) according to Claim 3, in which said second mirrors (122d, 122g) of the two optical assemblies (110d, 110g) are contiguous.

5. An optical system (100) according to Claim 4, in which the contiguous edge (124) of said second mirrors (122d, 122g) is placed approximately in the optical axis (Z) of the image acquisition module (190).

6. An optical system (100) according to one of the preceding claims, in which the optical paths (TOd, TOg) comprise optical output paths (118d, 118g) striking said image acquisition module (190), which run parallel to one another and to the optical axis (Z) of the image acquisition module.

7. An optical system (100) according to one of the preceding claims, comprising, at the level of the two input zones (112d, 112g) of the optical transmission means (110d, 110g) to be placed opposite each eye (22d, 22g), a means (140) for polarizing the light signals entering the system at the level of the input zones.

8. An optical system (100) according to one of the preceding claims, comprising infrared illumination means (130d, 130g) for directly illuminating in each case one of the two eyes (22d, 22g), said illumination means functioning with continuous emission.

9. An optical system (100) according to one of the preceding claims, in which the optical system (100) is hinge-mounted on a fixing arm (16).

10. A support device (10) for placing on the head of the individual (20) having two eyes (22, 22d, 22g) looking at an object in a plane of view (Δ), including an optical system (100) for tracking ocular movements according to one of the preceding claims, and in which a principal optical axis (Z) of the optical system (100) forms an angle (α) with the plane of view (Δ).

11. A device (10) according to Claim 10, in which the angle (α) between the principal optical axis (Z) and the plane of view (Δ) is between 20° and 45°, the optical system being placed below the plane of view (Δ).

12. A device (10) according to Claim 10 or 11, in which the optical system (100) is mounted on a fixing arm (16), articulated relative to a support structure (12), in such a way as to make variable the angle (α) between the principal optical axis (Z) and the plane of view (Δ).
